# EUROPÄISCHE PATENTSCHRIFT

(11) **EP 2 462 918 B1**
(45) Veröffentlichungstag und Bekanntmachung des Hinweises auf die Patenterteilung: **06.12.2017**
(21) Anmeldenummer: 11159142.6
(22) Anmeldetag: 22.03.2011
(51) Int. Cl.: A61K 8/31, A61K 8/81, A61K 8/891, A61K 8/92, A61Q 5/06, A61Q 5/12

(54) **Haarkonditioniermittel mit formgebenden Eigenschaften**
Hair conditioner with styling properties
Agents de conditionnement capillaire à propriétés fixantes

(30) Priorität: 16.07.2010 DE 102010027487
(43) Veröffentlichungstag der Anmeldung: 13.06.2012
(73) Patentinhaber: Beiersdorf AG, 20253 Hamburg (DE)
(72) Erfinder: Hugger, Anna, 22529, Hamburg (DE); Mahadeshwar, Anand, Pompton Lakes, NJ 07442 (US); Otto, Sebastian, 20253, Hamburg (DE)

(56) Entgegenhaltungen:
- EP-A1- 2 039 347
- WO-A1-01/01934
- WO-A1-2004/045564
- WO-A2-2006/106140
- FR-A1- 2 925 300
- US-A- 5 665 368
- US-A1- 2005 287 093
- DATABASE GNPD [Online] MINTEL; 31. März 2001 (2001-03-31), "PAINTS LIQUID LIPCOLOR SET", XP002724075, Database accession no. 10083396
- DATABASE GNPD MINTEL; 31. Juli 2007 (2007-07-31), "SPRAY WAX WITH COLOR PROTECTORS", XP002724076, Database accession no. 736935

## Beschreibung

Die Erfindung betrifft neue Haarkonditioniermittel mit formgebenden Eigenschaften.

Frisierbarkeit im Sinne der vorliegenden Schrift bedeutet die Möglichkeit die gewünschte Frisur einfach und schnell zu erstellen.

Alle Mengenangaben sind - sofern nicht anders angegeben - Massenprozente bezogen auf die Gesamtmasse der Zubereitung.

Pflegeperformance im Sinne der vorliegenden Schrift bedeutet die Pflegeleistung des Produktes. Diese zu verbessern heißt, die Pflegeparameter des Haares wie Entwirrbarkeit, Kämmbarkeit, Gleitvermögen, weiches und seidiges Haargefühl etc. zu verbessern.

"rinse off" Haarspülungen im Sinne der vorliegenden Schrift sind Pflegeprodukte welche nach gewisser Einwirkzeit auf den Haaren (bis zu 3 min) mit Wasser ausgespült werden.

Verteilbarkeit im Sinne der vorliegenden Schrift bedeutet die Möglichkeit das Produkt auf den Haaren gleichmäßig und einfach zu verteilen (die Haare sind mit dem Produkt gleichmäßig benetzt).

Gleitvermögen im Sinne der vorliegenden Schrift bedeutet die Möglichkeit nach der Produktverwendung mit den Fingern auf der Haaroberfläche ohne größeren Kraftaufwand zu gleiten. Die Skala wird von stumpf bis gut gleitend eingeteilt, Gleitvermögen kann im nassen und trockenen Haar beurteilt werden.

Haarstruktur im Sinne der vorliegenden Schrift bedeutet das Anfühlen des Haares, bewertet von Anwendern; die Skala reicht dabei von rauem bis glattem Haargefühl. Beim Bewerten wird darauf geachtet, wie einfach sich die einzeln Haarfasern zwischen den Fingern bewegen lassen.

Ausspülbarkeit im Sinne der vorliegenden Schrift bedeutet die Zeit, die in Sekunden vergeht, bis kein Produkt mehr an den Haaren zu fühlen ist.

Kämmbarkeit im Sinne der vorliegenden Schrift bedeutet wie leicht sich das Haar mit dem Kamm kämmen lässt; man unterscheidet zwischen Kämmbarkeit im nassen und trockenen Haar.

Panel Tests im Sinne der vorliegenden Schrift bedeutet das geschulte Panelisten verschiedene Produkte sensorisch bewerten, die Panelisten sind auf die Bewertung von definierten Parametern anhand von Standards trainiert. So können für jedes Produkt Leistungsprofile erstellt werden und Produkte untereinander verglichen werden.

Pflegeleistung im Sinne der vorliegenden Schrift bedeutet eine zusammengefasste Bewertung verschiedener Parameter wie Gleitvermögen, Haarstruktur, Kämmbarkeit, etc.; eine hohe Pflegeleistung ist insbesondere bei Produkten für geschädigtes Haar notwendig.

Sensorik im Sinne dieser Schrift umfasst alle fühlbaren und für den Anwender relevanten Eigenschaften des Produktes bei der Entnahme des Produktes aus der Packung, bestimmungsgemäßer Anwendung (Einmassieren in das Haar, Aufenthalt des Produktes im Haar, Ausspülen) sowie die während und nach der Anwendung fühlbaren Veränderungen an Haar, Kopfhaut und Fingern. Insbesondere umfasst "Sensorik" die Eigenschaften "Gehaltvoll", "Gleitvermögen", "Entwirrbarkeit", "Kämmbarkeit", "Haarstruktur", "Weich geschmeidig".

Die Verbraucher benutzen Haarspülungen um die sensorische Eigenschaften der Haare zu verbessern. Darunter ist insbesondere die Verbesserung von Parametern wie Kämmbarkeit, Geschmeidigkeit oder Haarstruktur (Glätte) zu verstehen. Die Verbraucher fassen diese Parameter unter dem Begriff Pflege zusammen. Pflegeeffekte haben ein Optimum und können bei einem zu hohem Pflegeniveau zu Beschwerung des Haares führen. Haare die "überpflegt" sind, können nur noch schwer geformt werden. Zur Verbesserung der Formbarkeit der Frisur werden häufig zusätzlich Styling Produkte verwendet. Nach der Anwendung von Styling Produkten geht allerdings die vom Verbraucher gewünschte "natürliche" Sensorik der Haare verloren.

Aus Verbrauchersicht ist es wünschenswert ein Produkt zu nutzen, welches gute Pflegeeigenschaften anbietet und gleichzeitig zur Verbesserung der Frisierbarkeit beiträgt. Darüber hinaus darf das Produkt keinen negativen Einfluss auf die Sensorik haben.

In rinse off Haarspülungen werden Silikone (z. B. Aminosilicone, Dimethicone), kationische Polymere (z. B. Guar, PQ 10), eingesetzt, um die Pflegeperformance zu erhöhen. Dabei lagern sich die Polymere und Silikone an der Haaroberfläche an und führen so zur Verbesserung der Geschmeidigkeit und der Glätte. Die Frisierbarkeit (Shape) des Haares wird dabei nur gering beeinflusst.

Styling Rohstoffe (z. B. PVP, VP/VA Copolymer, PQ-4) sind im Hair Styling Bereich sehr stark verbreitet und werden verwendet, um die gewünschte Frisur-/Haarform zu erreichen. Sie haben allerdings einen negativen Einfluss auf die Pflegeeigenschaften der Haare.

Ziel der Entwicklung war es ein Produkt zu entwickeln, dass eine gute Pflegeleistung hat ohne das Haar zu "überpflegen" und gleichzeitig die Formbarkeit des Haares (bzw. Frisierbarkeit) unterstützt.

Aus der DE 102006058390 A1 war ein Mittel zur Verformung keratinischer Fasern, enthaltend in einem kosmetisch akzeptablen Träger mindestens ein Haftklebstoff, ausgewählt aus Acrylsäureester-Copolymeren und Methacrylsäureester-Copolymeren, sowie die Verwendung entsprechender Haftklebstoffe in Stylingmitteln zur Induzierung oder Verbesserung der Remodellierbarkeit einer mit dem Stylingmittel erzeugten oder fixierten Frisur bekannt.

WO2008010190 offenbart eine im wesentlichen klare persönliche Pflegezubereitung mit 5 bis 50 % eines waschaktiven Tensides, 0.05 bis 20 % Wachspartikel mit einem Schmelzpunkt von 1000°C und einer mittleren Partikelgröße von weniger als 0.15 Mikrometer sowie wenigstens 20 % eines kosmetisch akzeptablen Mediums.

US20070183997 offenbart eine kosmetische Zubereitung mit Polymerpartikeln in einer Fettphase, die nicht mehr als 50 % flüchtiges Öl enthält und eine Fettphase, die wenigstens 5% eines wenig- oder nichtpolaren Öls enthält.

All dies konnte die Mängel des Standes der Technik nicht beseitigen.

Überraschend und für den Fachmann nicht vorhersehbar hat sich nun herausgestellt, dass Haarspülungen zum Ausspülen aus dem Haar, die eine Frisurfixierung erlauben enthaltend
A) als Silikon Dimethicone,
B) Wachse, bevorzugt Cera Microcristallina,
C) klebendes Polymer, wobei das klebende Polymer Polyisobuten ist,
D) Filmbildner, bevorzugt VP/Hexadecene Copolymer den Mängeln des Standes der Technik abhelfen. Dabei ist es bevorzugt, wenn das Silikon hochmolekular ist. Das Mengenverhältnis zwischen A) zu B) 2 zu 0,5 bis 0,5 zu 3 ist, ganz besonders bevorzugt 1 zu 2, das Mengenverhältnis zwischen A) zu C) 1 zu 4 bis 4 zu 0,5 ist, ganz besonders bevorzugt 3 zu 1, das Mengenverhältnis zwischen A) zu D) 1 zu 4 bis 4 zu 0,5 ist, ganz besonders bevorzugt 3 zu 1. Dabei ist es besonders bevorzugt, wenn die Wachse ausgesucht werden aus synthetischen Wachsen, besonders bevorzugt Paraffinwachs, Polyethylen-Wachs, Vinylpyrolidone Copolymeren, hydrolyziertem mikrokristallinem Wachs. Besonders bevorzugt ist es, wenn als klebendes Polymer Polyisobutene mit einem Molekulargewicht von 500 bis 100 000 g/mol verwendet wird. Die Erfindung umfasst auch die Verwendung der Mischung aus microcristallinem Wachs und Polyisobutene in einer Spülung zu Verbesserung der Frisierbarkeit des Haares. Die Erfindung umfasst weiter die Verwendung einer erfindungsgemäßen Haarspülung zur Verbesserung des Gleitvermögens, der Entwirrbarkeit, der Kämmbarkeit des Haares sowie der Haarstruktur und die Verwendung einer erfindungsgemäßen Zubereitung als leave on Produkt, z. B. als Haarbalm, leave in Kur zur Verbesserung der Sensorik des Haares z. B. Kämmbarkeit des Haares sowie des Haargefühls und der Haarstruktur.

Es wurden nun eine einzigartige Kombination aus folgenden vier Rohstoffen: Silikon, Wachs, Klebstoff und Filmbildner identifiziert. Mit dieser Kombination können die Vorteile eines klassischen rinse off Conditioner (z. B. Kämm- und Geschmeidigkeit) mit den positiven Eigenschaften der Stylingprodukte (z. B. Frisierbarkeit) kombiniert werden. Es wurden verschiedene Kombinationen und Mengenverhältnisse getestet. Die besten Ergebnisse wurden mit einer Kombination aus Silikon (Dimethicone), Wachs (Microcrystaline wax), Klebstoff (Polyisobuten) und Filmbildner (VP/Hexadecene Copolymer) erzielt. Einige der favorisierten Kombinationen sind in der nachfolgenden Tabelle aufgeführt.

Vorteilhaft können solche Zubereitungen zusätzlich UV- Filter, Polyole, Konservierungsmittel, hydrolysierte Proteine, Pflanzenextrakte, Parfümöle, und Antioxidantien sowie auch amphotere Tenside, Fettsäure Estern und deren Mischungen enthalten.
Muster 1,3 und 4 sind gemäß der Ansprüche.

### Beispiele für rinse off Conditioner

| INCI | w/w% | | | | | | | | |
|---|---|---|---|---|---|---|---|---|---|
| | Muster 1 | Muster 2 | Muster 3 | Muster 4 | Muster 5 | Muster 6 | Muster 7 | Muster 8 | Muster 9 |
| Microcrystalline wax⁽¹⁾ | 1 | 1 | 3 | 3 | 2 | 0 | 0,5 | 1 | 0 |
| Polyisobutene ⁽²⁾ | 3 | 3 | 0,5 | 1 | 1 | 3 | 0,5 | 0 | 0 |
| VP/Hexadecene copolymer ⁽³⁾ | 1 | 1 | 0,5 | 1 | 2 | 3 | 3 | 3 | 0 |
| Dimethicone ⁽⁴⁾ | 3 | 0 | 1,5 | 2 | 0 | 1,5 | 0 | 3 | 3 |
| Coco betaine ⁽⁵⁾ | 0,85 | 0,85 | 0,85 | 0,85 | 0,85 | 0 | 0 | 0,85 | 0,85 |
| Stearamidopropyl Dimethylamine⁽⁶⁾ | 2 | 2 | 2 | 2 | 2 | 0 | 2 | 2 | 2 |
| Cetyl Alcohol | 2 | 2 | 2 | 2 | 2 | 2 | 0 | 2 | 2 |
| Stearyl Alcohol | 4 | 4 | 4 | 4 | 4 | 4 | 4 | 4 | 4 |
| Lactic Acid | 1 | 1 | 1 | 1 | 1 | 1 | 1 | 1 | 1 |
| Preservative, antioxidant, pHadjustments, parfum | qs | qs | qs | qs | qs | qs | qs | qs | qs |
| Water | qs to 100 | qs to 100 | qs to 100 | qs to 100 | qs to 100 | qs to 100 | qs to 100 | qs to 100 | qs to 100 |

(1) - insbesondere von der Firma Paramelt,
(2) - insbesondere von der Firma Evonik,
(3) - insbesondere von der Firma Induchem
(4) - DC 200 Fluid Series
(5) - Dehyton AB 30 von der Firma Cognis
(6) - Tego Amid S18 von der Firma Evonik

Die aufgeführten Produkte wurden sowohl im Labor als auch in einem Spülungspanel durch Experten beurteilt. Die Bewertung fand im nassen Haar statt. Die Experten sind in der sensorischen Bewertung geschult. Im Vordergrund der Bewertung standen Parameter wie Verteilbarkeit, Gleitvermögen, Haarstruktur, Ausspülbarkeit und Kämmbarkeit. Die Ergebnisse der Panel Tests ließen eine Aussage zur Pflegeleistung und zur Sensorik der Produkte zu.

Im Anschluss an die Bewertung im nassen Haar wurde der Einfluss der Produkte auf die Formbarkeit der Haare im trockenen Haar untersucht. Dabei wurden die Produkte mit dem Standardverfahren angewendet und anschließend wurden die behandelten Strähnen im trockenen Haar beurteilt. Die Beurteilung wurde ebenfalls durch Experten vorgenommen. Es wurde darauf geachtet, ob die Verwendung der Produkte ein Einfluss auf die Formbarkeit hat. Dies könnte z. B. die Definition der Locken bei lockigen Strähnen oder ein zusätzlicher "Glättungseffekt" an glatten Strähnen (wie parallel die Einzel Haare "liegen") sein.

Die Ergebnisse zeigten, dass eine überlegene Sensorik und Pflegeleistung nur bei der optimalen Kombination aus Silikon (Dimethicone), Wachs (Microcrystaline wax), Klebstoff (Polyisobuten) und einen Filmbildner (VP/Hexadecene copolymer) erzielt wurde.

In der folgenden Tabelle sind die Ergebnisse des Paneltests dargestellt. Es wurde mit einer 5-er Skala bewertet, wobei 1 für schlechte und 5 für eine sehr gute Leistung steht. Das Muster 3 hat das beste Ergebnis geliefert. Am Paneltest haben 5 Experten teilgenommen.

| Formulation | Pflegeleistung | Form der Frisur | Stabilität |
|---|---|---|---|
| Muster 1 | 3 | 3 | 4 |
| Muster 2 | 2 | 2 | 4 |
| Muster 3 | 5 | 5 | 5 |
| Muster 4 | 4 | 4 | 5 |
| Muster 5 | 3 | 4 | 4 |
| Muster 6 | 4 | 3 | 4 |
| Muster 7 | 2 | 2 | 4 |
| Muster 8 | 4 | 3 | 3 |
| Muster 9 | 4 | 2 | 5 |

Das beste Ergebnis lieferte Formel 3. Das Mengenverhältnis zwischen Dimethicone-MicrocrystalineWax -Polyisobutene- VP/Hexadecene beträgt 1,5:3:0,5:0,5.

Im Anschluss wurde diese Formel (Muster 3) im Haarstudio gegen eine Benchmark getestet. Die Benchmark verfügt nicht über die "neue" Rohstoffkombination. Der Test wurde als Halbseiten Test auf trockenem/strapaziertem kaukasischem Haar mit n=10 Probanden durchgeführt.

| Pflegeparameter | Benchmark | Muster 3 (erfindungs-gemäß) |
|---|---|---|
| Verteilbarkeit | 6,6 | 6,7 |
| Gehalt (beim Verteilen) | 4,6 | 4,7 |
| Gleitvermögen (beim verteilen) | 6,0 | 6,3 |
| Gehalt beim ausspülen - sofort | 2,9 | 2,8 |
| Gleitvermögen beim ausspülen - sofort | 6,7 | 7,2 |
| Gleitvermögen beim ausspülen | 5,6 | 6,1 |
| Entwirrbarket (beim nassen Haar) | 5,7 | 6,4 |
| Kämmbarkeit (beim nassen Haar) | 6,5 | 7,2 |
| Gleitvermögen (beim nassen Haar) | 6,2 | 6,8 |
| Haarstruktur (nass -Haarlänge) | 6,6 | 7,2 |
| Haarstruktur (nass -Haarspitzen) | 5,0 | 6,0 |
| Frisurenfülle | 6,9 | 6,7 |
| Entwirrbarkeit (beim trockenen Haar) | 6,4 | 6,6 |
| Kämmbarkeit (beim trockenen Haar) | 7,0 | 7,5 |
| Gleitfähigkeit (beim trockenen Haar) | 6,7 | 6,9 |
| Haarstruktur (trocken - Haarlänge) | 7,5 | 7,8 |
| Haarstruktur (trocken - Spitzen) | 6,3 | 6,4 |

Das Ergebnis zeigt, dass alle Parameter mindestens auf gleichem oder höherem Niveau liegen. Parameter die die Pflegeeigenschaften beschreiben (z. B. Kämmbarkeit und Gleitvermögen) wurden signifikant besser bewertet.

In der nachfolgenden Tabelle ist die genaue Zusammensetzung der Benchmark dargestellt. Im Vergleich zu den getesteten Mustern 1 bis 9 enthält die Benchmark keinen Filmbildner.

| INCI | w/w % |
|---|---|
| Microcrystalline wax ⁽¹⁾ | 3,5 |
| Polyisobutene ⁽²⁾ | 1 |
| VP/Hexadecene copolymer ⁽³⁾ | 0 |
| Dimethicone ⁽⁴⁾ | 3 |
| Coco betaine ⁽⁵⁾ | 0,85 |
| Stearamidopropyl Dimethylamine ⁽⁶⁾ | 2 |
| Cetyl Alcohol | 2 |
| Stearyl Alcohol | 4 |
| Lactic Acid | 1 |
| Preservative, antioxidant, pH- adjustments, parfum | qs |
| Water | qs to 100 |

### Beispiele für Leave on Conditioner

Diese Kombination aus Silikon, Wachs, Klebstoff und Filmbildner ist auch für die anderen Arten von Pflegeprodukten geeignet (z. B. leave on Spülung, Balm, Creme, etc...).

| INCI | w/w % | | | |
|---|---|---|---|---|
| | Muster 1 | Muster 2 | Muster 3 | Muster 4 |
| Microcrystalline wax⁽¹⁾ | 1 | 0,5 | 3 | 2 |
| Polyisobutene ⁽²⁾ | 1 | 2 | 0,5 | 0,5 |
| VP/Hexadecene copolymer ⁽³⁾ | 1 | 2 | 0,5 | 0,5 |
| Dimethicone⁽⁴⁾ | 2 | 0 | 0,5 | 0,5 |
| Distearoylethyl Hydroxyethylmonium Methosulfate + Cetearyl Alcohol ⁽⁵⁾ | 2 | 2 | 2 | 2 |
| Palmitamidopropyltrimonium Chloride + Propylene Glycol⁽⁶⁾ | 1 | 1 | 1 | 1 |
| Hydroxypropyl Methylcellulose ⁽⁷⁾ | 0,25 | 0,25 | 0,25 | 0,25 |
| Cetearyl Alcohol | 2,5 | 2,5 | 2,5 | 2,5 |
| Glycerin | 8,5 | 8,5 | 8,5 | 8,5 |
| Preservative, antioxidant, pH- adjustments, parfum | qs | qs | qs | qs |
| Water | qs to 100 | qs to 100 | qs to 100 | qs to 100 |

| | | | | |
|---|---|---|---|---|
| (1) - insbesondere von der Firma Paramelt, (2) - insbesondere von der Firma Evonik, (3) - insbesondere von der Firma Induchem (4) - DC 200 Fluid Series (5) - Dehyquart F 75 von der Firma Cognis (6) - Varisoft PATC von der Firma Evonik (7) - Tylose E 707002 von der Firma Tylose SE | | | | |

## Patentansprüche

1. Haarspülungen zum Ausspülen aus dem Haar, die eine Frisurfixierung erlauben enthaltend
A) als Silikon Dimethicone,
B) Wachse, bevorzugt Cera Microcristallina,
C) klebendes Polymer, wobei das klebende Polymer Polyisobuten ist,
D) Filmbildner, bevorzugt VP/Hexadecene Copolymer und wobei
das Mengenverhältnis zwischen A) zu B) 2 zu 0,5 bis 0,5 zu 3 beträgt, ganz besonders bevorzugt 1 zu 2; das Mengenverhältnis zwischen A) zu C) 1 zu 4 bis 4 zu 0,5. beträgt, ganz besonders bevorzugt 3 zu 1 und das Mengenverhältnis zwischen A) zu D) 1 zu 4 bis 4 zu 0,5 beträgt, ganz besonders bevorzugt 3 zu 1.

2. Spülung nach Patentanspruch 1, **dadurch gekennzeichnet, dass** das Silikon hochmolekular ist.

3. Spülung nach einem der vorangehendenden Patentansprüche, **dadurch gekennzeichnet, dass** die Wachse ausgesucht werden aus synthetischen Wachsen, besonders bevorzugt Paraffinwachs, Polyethylen-Wachs, Vinylpyrolidone Copolymeren, hydrolyziertem mikrokristallinem Wachs.

4. Spülung nach einem der vorangehendenden Patentansprüche, **dadurch gekennzeichnet, dass** als klebendes Polymer Polyisobutene mit einem Molekulargewicht von 500 bis 100 000 g/mol verwendet wird.

5. Verwendung von Haarspülungen nach einem der vorangehenden Patentansprüche zur Verbesserung des Gleitvermögens, der Entwirrbarkeit, der Kämmbarkeit des Haares sowie der Haarstruktur.

6. Verwendung von Haarspülungen nach einem der vorangehenden Patentansprüche als leave on Produkt, z. B. als Haarbalm, leave in Kur zur Verbesserung der Sensorik des Haares z. B. Kämmbarkeit des Haares sowie das Haargefühl und die Haarstruktur.

## Claims

1. Hair conditioners for rinsing out of hair, which allow fixing of a hair style, comprising
A) dimethicone as silicone
B) waxes, preferably microcrystalline cera,
C) adhesive polymer, wherein the adhesive polymer is polyisobutene,
D) film former, preferably VP/hexadecene copolymer and wherein
the ratio of A) to B) is from 2:0.5 to 0.5:3, especially preferably 1:2; the ratio of A) to C) is from 1:4 to 4:0.5, especially preferably 3:1 and the ratio of A) to D) is from 1:4 to 4:0.5, especially preferably 3:1.

2. Conditioner according to Claim 1, **characterized in that** the silicone is high molecular weight.

3. Conditioner according to either of the preceding patent claims, **characterized in that** the waxes are selected from synthetic waxes, particularly preferably paraffin wax, polyethylene wax, vinylpyrrolidone copolymers, hydrolysed microcrystalline wax.

4. Conditioner according to any of the preceding patent claims, **characterized in that** the adhesive polymer used is polyisobutene having a molecular weight of 500 to 100 000 g/mol.

5. Use of hair conditioners according to any of the preceding patent claims for improving the slide, the detanglability, the combability of hair and also the hair structure.

6. Use of hair conditioners according to any of the preceding patent claims as a leave-on product, e.g. as hair balm, leave-in treatment for improving the sensory properties of hair, e.g. combability of hair and also hair feel and hair structure.

## Revendications

1. Agents de rinçage destinés à être éliminés par rinçage des cheveux, qui permettent une fixation de la coiffure, contenant
A) comme silicone du diméthicone
B) des cires, de préférence de la Cera Microcristallina
C) un polymère adhésif, le polymère adhésif étant le polyisobutène,
D) des agents filmogènes, de préférence un copolymère de VP/hexadécène,
le rapport pondéral de A) à B) étant de 2:0,5 à 0,5:3, de manière tout particulièrement préférée de 1:2 ; le rapport pondéral de A) à C) étant de 1:4 à 4:0,5, de manière tout particulièrement préférée de 3:1 et le rapport pondéral de A) à D) étant de 1:4 à 4:0,5, de manière tout particulièrement préférée de 3:1.

2. Agent de rinçage selon la revendication 1, **caractérisé en ce que** le silicone présente un haut poids moléculaire.

3. Agent de rinçage selon l'une quelconque des revendications précédentes, **caractérisé en ce que** les cires sont choisies parmi les cires synthétiques, de manière particulièrement préférée la cire de paraffine, la cire de polyéthylène, les copolymères de vinylpyrrolidone, la cire microcristalline hydrolysée.

4. Agent de rinçage selon l'une quelconque des revendications précédentes, **caractérisé en ce qu'**on utilise, comme polymère adhésif, des polyisobutènes présentant un poids moléculaire de 500 à 100.000 g/mole.

5. Utilisation des agents de rinçage pour cheveux selon l'une quelconque des revendications précédentes pour améliorer le pouvoir glissant, l'aptitude au démêlage, au peignage des cheveux ainsi que la structure des cheveux.

6. Utilisation des agents de rinçage selon l'une quelconque des revendications précédentes comme produits "leave on", par exemple comme baume pour cheveux, comme cure "leave in" pour améliorer les propriétés sensorielles des cheveux, par exemple l'aptitude au peignage des cheveux ainsi que le toucher des cheveux et la structure des cheveux.
